(19) Europäisches Patentamt
European Patent Office
Office européen des brevets

(11) EP 0 629 614 B1

# (12) EUROPÄISCHE PATENTSCHRIFT

(45) Veröffentlichungstag und Bekanntmachung des Hinweises auf die Patenterteilung:
**23.08.2000 Patentblatt 2000/34**

(51) Int. Cl.[7]: **C07D 207/404**, C07D 207/408

(21) Anmeldenummer: **94107839.6**

(22) Anmeldetag: **20.05.1994**

## (54) Substituierte Bernsteinsäureimide

Substituted succinimides

Succinimides substitués

(84) Benannte Vertragsstaaten:
**DE ES FR GB IT NL**

(30) Priorität: **27.05.1993 DE 4317651**

(43) Veröffentlichungstag der Anmeldung:
**21.12.1994 Patentblatt 1994/51**

(73) Patentinhaber: **Clariant GmbH**
**65929 Frankfurt am Main (DE)**

(72) Erfinder:
- **Dönges, Reinhard, Dr.**
**D-65812 Bad Soden (DE)**
- **Ehrler, Rudolf, Dr.**
**D-65439 Flörsheim (DE)**
- **Helwerth, Rainer, Dr.**
**D-65760 Eschborn (DE)**

(56) Entgegenhaltungen:

| | |
|---|---|
| **US-A- 3 184 474** | **US-A- 3 272 746** |
| **US-A- 3 324 033** | **US-A- 4 102 798** |

- **CARBOHYDRATE RESEARCH Bd. 52 , 1976 Seiten 49 - 61 Y. ISHIDO ET AL. 'Reactions of D-mannitol carbonate derivatives catalyzed by tetraethylammonium bromide'**
- **AZERB. KHIM. ZH. Nr. 2 , 1986 Seiten 119 - 122 A.B. ABDINOVA ET AL. 'Inhibition activity of unsaturated nitrogen-containing hydroxyethers'**
- **PRISADKI SMAZ. MASLAM Bd. 5 , 1978 Seiten 133 - 137 Z.E. ALIEV ET AL. 'Studies of the effect of some organic compounds on antifouling and anticorrosion properties of heavy fuels'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7 (HRS)/3.6

## Beschreibung

Nichtionische Tenside (siehe H. ueren, H. Großmann; Grenzflächenaktive Substanzen S. 83ff., Verlag Chemie 1971) sind heute in fast allen Wasch-und Reinigungsmitteln enthalten. Sie zeichnen sich vor allem durch gute Hautverträglichkeit und Stabilität gegen Wasserhärte aus.
Die wirtschaftlich größte Bedeutung unter den nichtionischen Tensiden haben die Polyalkylenoxid-Derivate erlangt. Man erhält sie durch Anlagerung von Alkylenoxiden, wie Ethylen- oder Propylenoxid, an Fettalkohole, Fettamine, Carbonsäuren oder Carbonsäureamide. Der hydrophile Teil des Tensids ist eine Polyetherkette. Über die Anzahl der addierten Ethermoleküle läßt sich die Hydrophilie des Tensids in weiten Grenzen einstellen. Ein Nachteil bei der großtechnischen Herstellung dieser Verbindungen ist die hohe Toxizität und das hohe Gefahrenpotential der Rohstoffe, insbesondere des Ethylenoxids, wodurch aufwendige sicherheitstechnische Maßnahmen bei der Produktion erforderlich werden.

Aminoxide, Sulfoxide und Phosphinoxide mit langkettigen Alkylresten sind ebenfalls als nichtionische Tenside beschrieben. Der stark dipolare Charakter der Heteroatom-Sauerstoff-Bindung bildet hier das hydrophile Zentrum. Ihre wirtschaftliche Bedeutung als Tenside ist jedoch gering.

Ein weiteres großes Feld im Bereich der nichtionischen Tenside stellen die Verbindungen dar, deren hydrophiler Teil durch Kohlenhydrate (siehe G. Gawalek, Tenside, Akademie-Verlag Berlin 1975, S. 274ff.) gebildet werden. Von großem wirtschaftlichem Interesse sind dabei die Alkyloligoglucoside, die aus den leicht zugänglichen Rohstoffen Fettalkohol und Glucose oder Stärke hergestellt werden können. Sie gelten, wie die meisten Kohlenhydrattenside, als besonders hautfreundlich und gut biologisch abbaubar. Die handelsüblichen Alkyloligoglucoside werden durch Fischer-Glycosidierung oder durch Umglycosidierung kurzkettiger Alkylglycoside mit Fettalkoholen gewonnen. Herstellungsbedingt handelt es sich um Gemische verschiedener kurzkettiger Oligoglucoside.

Zu den am längsten bekannten Kohlenhydrat-Tensiden gehören die Zuckerester. Sie werden durch Umesterung der Fettsäuremethylester mit Kohlenhydraten, meist Saccharose, erhalten (Snell et al. Ind. Eng. Chem. 48, 1459 (1956)).

Durch Kondensation von Fettsäuren mit Alditolen, wie Sorbit, erhält man je nach Bedingungen Alditolester (GB-A-872 507), Anhydroalditol- und Dianhydroalditolester (US-A-2 322 820). Generell ist jedoch die Anwendungsbreite von nichtionischen Tensiden auf Esterbasis durch die leichte Verseifbarkeit der Estergruppe stark eingeschränkt.

Die Kondensation von Alkylglycaminen und Fettsäuren zu N-Acylglycaminen, bei denen der Zuckerrest über eine Aminogruppe an die Fettsäure gebunden ist, ist Gegenstand der US-A-1 985 424. Diese langkettigen Polyhydroxyamide finden als Textilhilfsmittel Verwendung.

Wendet man bei der Kondensation höhere Temperaturen an, so bilden sich unter Wasseraustritt die entsprechenden Anhydro- und Dianhydroglycamide (US-A-2 993 887). Auch sie werden als waschaktiv beschrieben.

US-A-3 018 247, US-A-3 018 250 und US-A-3 018 291 offenbaren Alkenylbernsteinsäureimide, die durch Umsetzung von Alkenylbernsteinsäure bzw. deren Anhydrid und einem Polyamin (z.B. Tetraethylenpentamin) erhalten werden sowie deren Verwendung als Dispergiermittel für Schmieröle.

US-A-4 102 798 beschreibt die Umsetzung von Alkenylbernsteinsäureanhydriden mit monosubstituierten 2-Aminoethanolen.

US-A-4 102 798 beschreibt die Umsetzung von Alkenylbernsteinsäureanhydriden mit monosubstituierten 2-Aminoethanolen.

Carbohydrate Research 52, 49-61 (1976) beschreibt Reaktionen von 1,2;3,4-di-O-isopropyliden-D-mannitol-5,6-carbonat mit Succinimid, wobei N-(1-deoxy-3,4;5,6-di-O-isopropyliden-D-mannitol-1-yl)-succinimid entsteht.

Azerb. Khim. Zh., Nr. 2, 1986, pp. 119-122 beschreibt N-substituierte Bernsteinsäureimide, die Allyloxypropanol-2-Reste tragen.

Prisadki Smaz. Maslam, Bd. 5, 1978, pp. 133-137 beschreibt N-substituierte Bernsteinsäureimide, die Oxyethyltetrapropenyl- und Benzyltetrapropenylreste tragen.

US-A-3 184 474 nennt Umsetzungsprodukte von Alkenylbernsteinsäuren bzw. deren Anhydriden mit einem Gemisch aus mehrwertigen Alkoholen (z.B. Glykolen, Aminoalkoholen) und Polyaminen (z.B.Dimethylaminomethylamin, Tetrapropylenpentamin) und deren Verwendung als Korrosionsschutzmittel in Schmierölen.

Ausgehend von dem oben beschriebenen Stand der Technik ist es die Aufgabe der vorliegenden Erfindung Bernsteinsäureimide auf Basis von primären Zuckeraminen zur Verfügung zu stellen.

Gegenstand der Erfindung sind Bernsteinsäureimide der Formel I

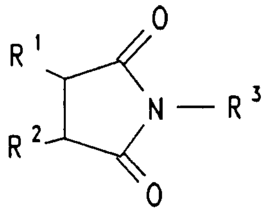

( I )

in der

$R^1$ und $R^2$ unabhängig voneinander Wasserstoff, $C_1$-$C_{24}$-Alkyl, $C_2$-$C_{24}$-Alkenyl, $C_6$-$C_{12}$-Cycloalkyl, $C_6$-$C_{12}$-Cycloalkenyl, $C_6$-$C_{12}$-Aryl, $C_1$-$C_{24}$-Alkoxy, $C_1$-$C_{24}$-Acyl, $C_1$-$C_{24}$-Acyloxy, eine Hydroxy-, Amino- oder Sulfonsäuregruppe oder einen $C_1$-$C_{24}$-Dialkylamino-, $C_1$-$C_{24}$-Acylamino-, $C_1$-$C_{24}$-Alkylsulfat-, $C_1$-$C_{24}$-Alkylsulfonat-, $C_1$-$C_{24}$-Alkylsulfit-, $C_1$-$C_{24}$-Alkylsulfoxid-, $C_1$-$C_{24}$-Alkoxycarbonylrest darstellen und $R^3$ ein Polyhydroxyalkylrest der allgemeinen Formel

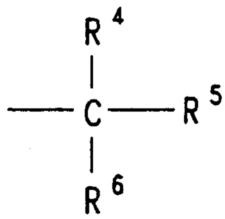

( II )

wobei

$R^4$ = $-(CHOR^7)_n-H$,
$R^5$ = $-(CHOR^7)_m-H$,
$R^6$ = $-(CHOR^7)_o-H$ mit $n + m + o = 2\text{-}6$ und
$R^7$ = H oder ein glycosidisch gebundenes Kohlenhydrat aus der Reihe der reduzierenden Zucker bedeuten.

Die Verbindungen der Formel I werden durch Umsetzung von Carbonsäuren der Formel IIa und/oder Carbonsäureanhydriden der Formel IIb

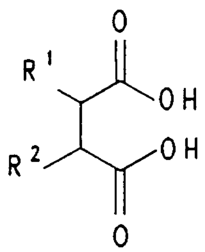

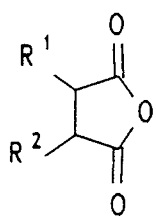

mit Aminoverbindungen der Formel III

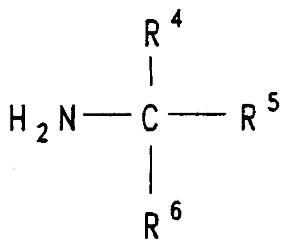

in denen die Reste $R^1$, $R^2$, $R^3$, $R^4$, $R^5$ und $R^6$ die obengenannte Bedeutung besitzen, erhalten.

Bevorzugte Verbindungen der Formel IIa/IIb sind Verbindungen, in denen

$R^1$ und $R^2$ Wasserstoff oder $C_6$-Cycloalkenyl,
$R^1$ Wasserstoff und $R^2$ $C_1$-$C_{24}$-Alkyl oder $C_2$-$C_{24}$-Alkenyl,
$R^1$ Wasserstoff und $R^2$ eine Hydroxygruppe und
$R^1$ einen $C_1$-$C_{24}$-Acylaminorest und $R^2$ Wasserstoff darstellen.

Beispielhaft seien genannt: Bernsteinsäureanhydrid, $C_1$-$C_{24}$-Alkylbernsteinsäureanhydrid, $C_2$-$C_{24}$-Alkenylbernsteinsäureanhydrid, $C_6$-Cycloalkenylbernsteinsäureanhydrid und Äpfelsäure.

Bei den Verbindungen der Formel III handelt es sich um Aminopolyole, die üblicherweise durch reduktive Aminierung der entsprechenden Aldosen, Ketosen, Di- oder Oligosaccharide erhalten werden, wobei Glucose, Galaktose, Fructose, Maltose, Lactose und Stärkehydrolysate bevorzugt eingesetzt werden. Ein Verfahren zur Herstellung von Aminopolyolen durch reduktive Aminierung von Aldosen in Gegenwart von Ammoniak oder Hydrazin wird in US-A-2 830 983 beschrieben. Die DE-A-3 625 931 nennt ein Verfahren zur reduktiven Aminierung von Ketosen, Di- und Oligosacchariden. Weitere geeignete Aminopolyole sind Verbindungen mit einer primären Aminogruppe und mindestens drei Hydroxylgruppen, z.B. Tris-hydroxyalkylaminomethan.

Ein weiterer Gegenstand der Erfindung ist ein Verfahren zur Herstellung der Bernsteinsäureimide der Formel I umfassend die Maßnahmen:

- Einbringen des Aminopolyols der Formel III in ein Suspensions- oder Lösungsmittel,
- Zugabe des Carbonsäureanhydrids IIa und/oder der Carbonsäure IIb, gegebenenfalls unter leichtem Erwärmen und
- Erhitzen dieses Gemisches auf eine Temperatur und über einen so langen Zeitraum bis das Gemisch weitgehend frei an Suspensions-oder Lösungsmittel bzw. gebildetem Reaktionswasser ist und das Reaktionsgemisch einen Basenstickstoffgehalt kleiner 0,1 Gew.-% aufweist.

Geeignete Suspensions- oder Lösungsmittel stellen Wasser, Alkohol oder Wasser/Alkohol-Gemische dar. Als Alkohole eignen sich besonders niedere aliphatische Alkohole, wie Methanol, Ethanol, Propanole, Butanole und/oder Pentanole.

Das erfindungsgemäße Herstellungsverfahren wird beispielhaft an der Herstellung von Alkenylbernsteinsäureimiden der Formel I erläutert. Zu ihrer Synthese kann man von großtechnisch herstellbaren Alkenylbernsteinsäureanhydriden ausgehen. Diese sind wiederum durch Umsetzung von Olefinen mit Maleinsäureanhydrid gut zugänglich. Zum anderen benötigt man die vorstehend beschriebenen Aminopolyole.

Das Aminopolyol wird in Wasser, einem niederen Alkohol oder einem Gemisch daraus gelöst bzw. suspendiert. Als Lösemittel sind insbesondere Methanol und Butanol geeignet. Bei Anwesenheit von Wasser empfiehlt sich die Zugabe eines tertiären Amins als Hilfsbase. Besonders Triethylamin ist geeignet, da es sich leicht zusammen mit den anderen Lösungsmitteln abdestillieren und im Folgeansatz wieder einsetzen läßt.

In der ersten Reaktionsstufe wird bei leicht erhöhter Temperatur, im allgemeinen im Bereich zwischen 20 bis 80°C, bevorzugt 50 bis 70°C, das Alkenylbernsteinsäureanhydrid in die Lösung (oder Suspension) des Aminopolyols getropft, wobei sich das Monoamid bildet. Man rührt eine gewisse Zeit nach und beginnt dann bei erhöhter Temperatur das Lösungs- bzw. Suspensionsmittel abzudestillieren. Die erhöhte Temperatur liegt im allgemeinen bei 100 bis 160°C. Ist kein Lösungsmittel mehr vorhanden, steigert man die Temperatur bis auf höchstens 190°C und führt in der Schmelze unter Wasseraustritt im leichten Stickstoffstrom die Kondensation zum Imid durch. Die Reaktion ist beendet, wenn eine Probe des Reaktionsproduktes, gelöst in Essigsäure und titriert mit 0.1 N Perchlorsäure, einen Basenstickstoffgehalt von kleiner 0.1 % aufweist. Die Bildung des Bernsteinsäureimids läßt sich durch Infrarotspektroskopie nachweisen. Eine starke Bande bei ca. 1700 $cm^{-1}$ und eine schwächere bei ca. 1780 $cm^{-1}$ sind für eine Imid-Struktur charakteristisch. Unter der Voraussetzung, daß die Lösemittel und die Hilfsbase wieder eingesetzt werden, fallen bei der Herstellung keine Nebenprodukte an.

Ausgehend von reinen (destillierten) Alkenylbernsteinsäureanhydriden lassen sich die erfindungsgemäß hergestellen Bernsteinsäureimide nach Kristallisation in reiner Form isolieren. Sie können mittels $^1$H-NMR, $^{13}$C-NMR und Hochdruck-Flüssigkeitschromatographie (HPLC) charakterisiert werden. Erwartungsgemäß fällt bei der Kondensation eines chiralen, racemischen Alkenylbernsteinsäureanhydrids mit einem enantiomerenreinen Aminopolyol, wie Glucamin, ein Produktgemisch aus zwei Diastereomeren im Verhältnis von etwa 1:1 an. Anhand von HPLC-Chromatogrammen und teilweise im $^{13}$C-NMR-Spektrum ist dies an Doppelpeaks zu erkennen.

Die erfindungsgemäßen Verbindungen finden in verschiedenen Anwendungsbereichen Verwendung und eignen sich zum Beispiel als Waschrohstoffe. Des weiteren lassen sich diese Verbindungen mit Wasser zu hochkonzentrierten, technisch gut handhabbaren Tensidlösungen verrühren. Falls erforderlich kann das Produkt mit Wasserstoffperoxid gebleicht werden. Es hat sich gezeigt, daß diese Tensidlösungen sich darüberhinaus, ebenso wie die

erfindungsgemäßen Bernsteinsäureimide, als Korrosionsschutzmittel eignen. Üblicherweise werden bei dieser Verwendung 10 - 60 Gew.-% einer Stickstoffbase, bevorzugt tertiäre Amine, insbesondere Trialkanolamine, bezogen auf das Gewicht der Lösung, zugesetzt, wobei der pH-Wert derartiger wäßriger Lösungen größer 7, bevorzugt 8 bis 12 ist.

Herstellungsbeispiele:

Beispiel 1

Octenyl-N-(1-deoxyglucityl)-bernsteinsäureimid

In einem 1l-4-Halskolben mit Rührer, Rückflußkühler, Innenthermometer, Tropftrichter und Stickstoffeinlaß werden 90,5 g Glucamin in 300 ml wassergesättigtem Butanol vorgelegt. Bei 60°C Innentemperatur werden 102,8 g Octenylbernsteinsäureanhydrid (technisch) über 50 min zugetropft. Nach weiteren 60 min Rühren bei 60°C wird der Rückflußkühler durch eine Destillationsbrücke ersetzt. Unter leichtem Stickstoffstrom wird Wasser/Butanol abdestilliert. Die Sumpftemperatur wird bis auf 150°C gesteigert. Nach 3 h beträgt der Basenstickstoffgehalt des Reaktionsproduktes nur noch 0.2 %. Nach Abkühlen werden 167 g des Produktes erhalten .

Beispiel 2:

Tetrapropenyl-N-(1-deoxyglucityl)-bernsteinsäureimid.
(Apparatur wie in Beispiel 1)

271,5 g Glucamin werden in 700 ml wassergesättigtem Isobutanol vorgelegt. Innerhalb von 1 h läßt man 443,4 g Tetrapropenylbernsteinsäureanhydrid (technisch) bei 60°C Innentemperatur zulaufen. Man rührt noch 1 h bei 60°C nach und destilliert dann die Lösungsmittel ab. Die am Ende des Destillationsvorganges erreichte Temperatur von 160°C (Sumpf) wird noch 2 h lang gehalten. Die Schmelze wird auf kalte Bleche gegossen und nach dem Erstarren granuliert. Im Infrarot-Spektrum zeigt das Produkt die für Imide charakteristischen Banden bei 1695 $cm^{-1}$ (sehr stark) und 1770 $cm^{-1}$ (schwach). Die Säurezahl des Produkts beträgt 20 mg KOH/g, der Basenstickstoffgehalt 0.06 %.

Beispiel 3:

Tripropenyl-N-(1-deoxyglucityl)-bernsteinsäureimid
(Apparatur und Durchführung wie in Beispiel 2)

Es werden 386,3 g Tripropenylbernsteinsäureanhydrid (technisch) eingesetzt.
Kenndaten des Produkts:

IR-Spektrum (KBr): 1695 $cm^{-1}$ (stark), 1770 $cm^{-1}$ (schwach).
Säurezahl: 22 mg KOH/g.
Basenstickstoff: < 0.1 %.

Beispiel 4:

Dodecenyl-N-(1-deoxyglucityl)-bernsteinsäureimid.
(Apparatur wie in Beispiel 1)

181 g Glucamin werden in 600 ml wassergesättigtem Isobutanol und 101 g Triethylamin suspendiert. Innerhalb 1 h werden 266 g Dodecenylbernsteinsäureanhydrid (technische Ware, destilliert) zugetropft. Es entsteht eine klare Lösung, die noch eine weitere Stunde bei 60°C gehalten wird. Bei 130°C Badtemperatur werden Isobutanol, Wasser und Triethylamin abdestilliert, darauf wird die Temperatur auf 165°C gesteigert. Unter einem leichten Stickstoffstrom wird 3 h lang kondensiert, wobei noch geringe Mengen an Lösungsmittel und Triethylamin übergehen. Der Basenstickstoffgehalt beträgt nur noch 0.09 %. Das Rohprodukt besteht nach HPLC-Analyse zu 85 % aus dem gewünschten Produkt. Der erkaltete Reaktionsansatz wird aus 400 ml Isopropanol umkristallisiert. Man erhält 355.5 g (84 % d. Th.) farblose Kristalle mit einem Schmelzpunkt von 100-105°C. Die Hydroxylzahl beträgt 652. Dies entspricht einem Molekulargewicht von 430 (Theorie: 429).

$^1$H-NMR-Spektrum (399.65 MHz, DMSO-$d_6$):
$\delta$= 0,9-1,4 (17H, Alkyl-H), 1,95-2,0 (4H, Allyl-H), 2,28; 2,71 (2H jew dd -$CH_2$-CO-), 2,84 (1H, R-CH-CO-), 3,2-3,7

(5H, -CHOH), 3,62; 3,85 (2H, N-$CH_2$), 4,3-4,8 (5H, -OH), 5,32; 5,50 (2H, CH=CH-).
$^{13}$C-NMR-Spektrum (100.4 MHz, DMSO-$d_6$):
$\delta$= 13,88 ($CH_3$), 22,04-33,2 (-$CH_2$-, R-CHR-CO-, R-$CH_2$-CO-), 41,59 (N-$CH_2$-), 63,26 (-$CH_2$OH), 68,96-71,69 (-CHOH, 7 Signale), 125,15; 125,7; 132,5; 133,4 (-CH=), 176,6; 176,7; 179,5; 179,6 (-CO-).
Kritische Mizellkonzentration (Ringtensiometer, 25°C): 40 ppm.

Herstellung einer Tensidlösung:

Beispiel 5:

Dodecenyl-N-(1-deoxyglucityl)-bernsteinsäureimid

In einem 1l-4-Halskolben mit Rührer, Rückflußkühler, Innenthermometer, Tropftrichter und Stickstoffeinlaß werden 90.5 g Glucamin in 300 ml wassergesättigtem Butanol vorgelegt. Bei 60°C Innentemperatur werden 133.0 g Dodecenylbernsteinsäureanhydrid (technisch) über 1.5 h zugetropft. Nach weiteren 30 min Rühren bei 60°C wird der Rückflußkühler durch eine Destillationsbrücke ersetzt. Unter leichtem Stickstoffstrom wird Wasser/Butanol abdestilliert. Die Sumpftemperatur wird bis auf 185°C gesteigert, die Kopftemperatur steigt bis 130°C an. Nach Erreichen von 185°C Sumpftemperatur wird noch eine Stunde lang bei dieser Temperatur gerührt, dann innerhalb 1.5 h auf 100°C abgekühlt. Es werden 140 g Wasser zugesetzt und homogen verrührt. Bei 90°C werden 5.0 g einer 35 gew.-%igen Wasserstoffperoxidlösung zur Bleichung zugesetzt und eine Stunde verrührt. Man erhält ein fließfähiges Tensidkonzentrat (60.2 %), das sich gut mit Wasser mischt und einen Kältetrübungspunkt von -7°C aufweist.
Anwendungstechnische Prüfung der Tensidlösung als Waschrohstoff:

Schaumvermögen nach Ross//Miles:
220/220 bei 0.1 % WAS
80/80 bei 0.006 % WAS

(WAS = Waschaktive Substanz)
Die Farbe der Tensidlösung wird mittels Jod-Farbzahl bestimmt. Die Iod-Farbzahl beträgt 32 (bei 40 % WAS).
Anwendungstechnische Prüfung der Tensidlösung als Korrosionsschutzmittel: 167g Octenyl-N-(1-deoxyglucityl)-bernsteinsäueimid aus Beispiel 2 werden mit 111 g Wasser homogen verrührt. Es resultiert ein braunes viskoses Tensidkonzentrat mit 60 % Feststoffgehalt. 35 Teile dieses Konzentrats werden mit 15 Teilen vollentsalztem Wasser und 50 Teilen Triethanolamin homogen vermischt.
Die Lösung wird nach DIN 51 360/II auf Korrosionsschutzeigenschaften geprüft:

| c (in 20° dH-Wasser) | Note |
|---|---|
| 2 % | 2 |
| 3 % | 1 |
| 4 % | 0 |
| 5 % | 0 |

Beispiel 6

N-(1-Deoxyglucityl)-bernsteinsäureimid (Bernsteinsäureglucimid).
Apparatur wie Beispiel 1.

90,5 g Glucamin werden in 300 ml Methanol vorgelegt. Bei 50°C werden unter Stickstoffatmosphäre portionsweise 50,0 g Bernsteinsäureanhydrid eingetragen. Man rührt 1 h bei 60°C nach, steigert dann die Badtemperatur langsam auf 130°C und destilliert dabei Methanol ab. Es entsteht eine klare Schmelze, die noch 3 h lang unter Rühren im Stickstoffstrom auf 165°C erhitzt wird. Das Rohprodukt hat dann einen Basenstickstoff-Gehalt von 0.07 %. Es wird in 100 ml Wasser heiß gelöst und bis zur beginnenden Trübung mit Ethanol versetzt. Es resultieren 108 g farblose Kristalle vom Schmp. 176-178°C, einer Hydroxyl-Zahl von 1032 mg/g (Theorie: 1077), Basenstickstoff 0.04 %. IR-Banden bei 1695 $cm^{-1}$ und 1775 $cm^{-1}$ (KBr) bestätigen die Imid-Struktur der Verbindung.

Beispiel 7

N-(1-Deoxyglucityl)-hydroxybernsteinsäureimid (Äpfelsäureglucimid).
Apparatur wie Beispiel 1.

45,3 g Glucamin werden in 125 ml Methanol vorgelegt. Man gibt 33,5 g Äpfelsäure zu und kocht 3 h am Rückfluß. Der Rückflußkühler wird gegen eine Destillationsbrücke ausgetauscht und das Methanol vollständig abdestilliert. Der Rückstand wird im Stickstoffstrom 3 h lang auf 175°C erhitzt. Nach dem Abkühlen resultiert ein braunes, glasartiges Produkt mit einem Basenstickstoffgehalt von 0.1 %. Im Infrarot-Spektrum zeigen die Absorptionsbanden bei 1700 $cm^{-1}$ und 1780 $cm^{-1}$ (Film) die Anwesenheit des Imids.

**Patentansprüche**

1. Bernsteinsäureimide der Formel I

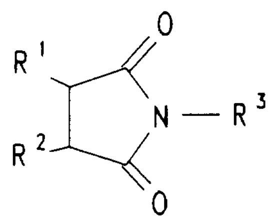

( I )

in der
$R^1$ und $R^2$ unabhängig voneinander Wasserstoff, $C_1$-$C_{24}$-Alkyl, $C_2$-$C_{24}$-Alkenyl, $C_6$-$C_{12}$-Cycloalkyl, $C_6$-$C_{12}$-Cycloalkenyl, $C_6$-$C_{12}$-Aryl, $C_1$-$C_{24}$-Alkoxy, $C_1$-$C_{24}$-Acyl, $C_1$-$C_{24}$-Acyloxy, eine Hydroxy-, Amino- oder Sulfonsäuregruppe oder einen $C_1$-$C_{24}$-Dialkylamino-, $C_1$-$C_{24}$-Acylamino, $C_1$-$C_{24}$-Alkylsulfat-, $C_1$-$C_{24}$-Alkylsulfonat-, $C_1$-$C_{24}$-Alkylsulfit-, $C_1$-$C_{24}$-Alkylsulfoxid-, $C_1$-$C_{24}$-Alkoxycarbonylrest darstellen, aber $R^1$ und $R^2$ nicht gleichzeitig Wasserstoff sind, und $R^3$ ein Polyhydroxyalkylrest der allgemeinen Formel

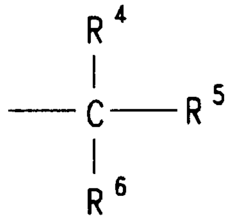

( I I )

ist, wobei

$R^4$ = -$(CHOR^7)_n$-H,
$R^5$= -$(CHOR^7)_m$-H,
$R^6$= -$(CHOR^7)_o$-H mit n + m + o = 2-6 und
$R^7$ = H oder ein glycosidisch gebundenes Monosaccharid aus der Reihe der reduzierenden Zucker sind.

2. Verfahren zur Herstellung der Bernsteinsäureimide der Formel I umfassend die Maßnahmen:

- Einbringen des Aminopolyols der Formel III

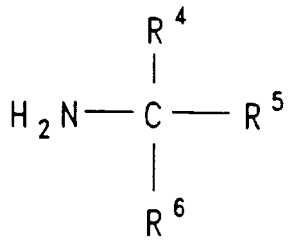

worin $R^3$, $R^4$ und $R^5$ die in Anspruch 1 angegebene Bedeutung besitzen, in ein Suspensions- oder Lösungsmittel,

- Zugabe der Carbonsäure IIa und/oder des Carbonsäureanhydrids IIb,

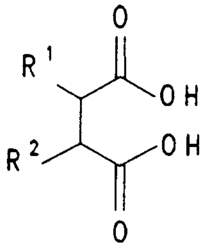

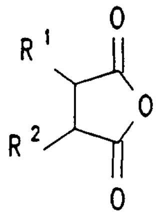

worin $R^1$ und $R^2$ die in Anspruch 1 angegebene Bedeutung besitzen, gegebenenfalls unter leichtem Erwärmen und

- Erhitzen dieses Gemisches auf eine Temperatur und über einen so langen Zeitraum bis das Gemisch weitgehend frei an Suspensions- oder Lösungsmittel bzw. gebildetem Reaktionswasser ist und das Reaktionsgemisch einen Basenstickstoffgehalt kleiner 0,1 Gew.-% aufweist.

**3.** Verfahren nach Anspruch 2, dadurch gekennzeichnet, daß als Suspensions- oder Lösungsmittel Wasser, Alkohol, bevorzugt niedere aliphatische Alkohole oder ein Wasser/Alkohol-Gemisch eingesetzt wird.

**4.** Verfahren nach Anspruch 2, dadurch gekennzeichnet, daß die Zugabe bei einer Temperatur zwischen 20 bis 80°C, bevorzugt 50 bis 70°C erfolgt.

**5.** Verfahren nach Anspruch 2, dadurch gekennzeichnet, daß das Gemisch zur Entfernung des vorhandenen Suspensions- bzw. Lösemittels auf eine Temperatur bis maximal 160°C und anschließend gegebenenfalls auf eine Temperatur bis maximal 190°C erhitzt wird.

**6.** Verfahren nach Anspruch 2, dadurch gekennzeichnet, daß das Gemisch über einen Zeitraum von bis zu 3 Stunden erhitzt wird.

**7.** Verwendung der Bernsteinsäureimide der Formel I nach Anspruch 1 als Waschrohstoff oder Korrosionsschutzmittel.

**8.** Wäßrige Lösung enthaltend Bernsteinsäureimide der Formel I nach Anspruch 1.

**9.** Wäßrige Lösung nach Anspruch 8, dadurch gekennzeichnet, daß sie 10 - 60 Gew.-% einer Stickstoffbase, bevorzugt tertiäre Amine enthält und einen pH-Wert größer 7 besitzt.

**10.** Verwendung der wäßrigen Lösung nach Anspruch 8 als Korrosionsschutzmittel.

## Claims

**1.** A succinimide of the formula I

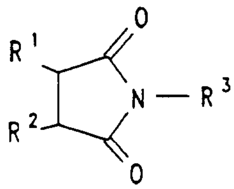

( I )

in which
$R^1$ and $R^2$, independently of one another, are hydrogen, $C_1$-$C_{24}$-alkyl, $C_2$-$C_{24}$-alkenyl, $C_6$-$C_{12}$-cycloalkyl, $C_6$-$C_{12}$-cycloalkenyl, $C_6$-$C_{12}$-aryl, $C_1$-$C_{24}$-alkoxy, $C_1$-$C_{24}$-acyl, $C_1$-$C_{24}$-acyloxy, a hydroxyl, amino or sulfo group or a $C_1$-$C_{24}$-dialkylamino, $C_1$-$C_{24}$-acylamino, $C_1$-$C_{24}$-alkyl sulfate, $C_1$-$C_{24}$-alkyl sulfonate, $C_1$-$C_{24}$-alkyl sulfite, $C_1$-$C_{24}$-alkyl sulfoxide or $C_1$-$C_{24}$-alkoxycarbonyl radical, but $R^1$ and $R^2$ are not simultaneously hydrogen, and $R^3$ is a polyhydroxyalkyl radical of the formula

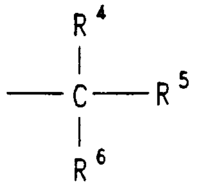

( I I )

in which

$R^4$ is $-(CHOR^7)_n-H$,
$R^5$ is $-(CHOR^7)_m-H$,
$R^6$ is $-(CHOR^7)_o-H$ where $n + m + o$ is 2-6, and
$R^7$ is H or a glycosidically attached monosaccaride selected from reducing sugars.

**2.** A process for preparing a succinimide of the formula I, comprising the steps of

- introducing the amino polyol of the formula III

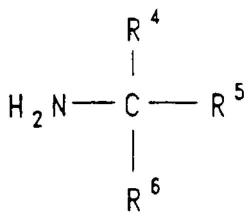

in which $R^3$, $R^4$ and $R^5$ have the meaning given in claim 1, into a suspendant or solvent,
- adding the carboxylic acid IIa and/or the carboxylic anhydride IIb,

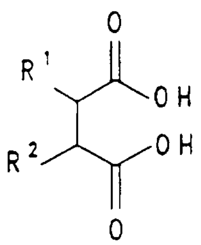

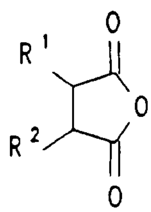

in which $R^1$ and $R^2$ have the meaning given in claim 1, if appropriate with slight heating, and

- heating this mixture at a temperature and over a sufficiently long period to make the mixture substantially free of suspendant or of solvent and the water of reaction formed and to bring the base nitrogen content of the reaction mixture down to less than 0.1 % by weight.

**3.** The process as claimed in claim 2, wherein the suspendant or solvent used is water, alcohol, preferably lower aliphatic alcohols, or a water/alcohol mixture.

**4.** The process as claimed in claim 2, wherein the addition takes place at a temperature of between 20 and 80°C, preferably 50 to 70°C.

**5.** The process as claimed in claim 2, wherein, in order to remove the suspendant or solvent present, the mixture is heated to a maximum temperature of 160°C and then, if desired, to a maximum temperature of 190°C.

**6.** The process as claimed in claim 2, wherein the mixture is heated over a period of up to 3 hours.

**7.** Use of a succinimide of the formula I as claimed in claim 1 as detergent base material or anti-corrosive.

**8.** An aqueous solution containing a succinimide of the formula I as claimed in claim 1.

**9.** An aqueous solution as claimed in claim 8, which contains 10-60 % by weight of a nitrogen base, preferably a tertiary amine, and has a pH of greater than 7.

**10.** Use of an aqueous solution as claimed in claim 8 as anticorrosive.

## Revendications

**1.** Imides de l'acide succinique de formule I

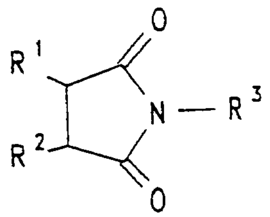

(I)

dans laquelle

$R^1$ et $R^2$ représentent, indépendamment l'un de l'autre, des atomes d'hydrogène, des groupes alkyle en $C_1$-$C_{24}$, alcényle en $C_2$-$C_{24}$, cycloalkyle en $C_6$-$C_{12}$, cycloalcényle en $C_6$-$C_{12}$, aryle en $C_6$-$C_{12}$, alkoxy en $C_1$-$C_{24}$, acyle en $C_1$-$C_{24}$, acyloxy en $C_1$-$C_{24}$, un groupe hydroxy, amino ou acide sulfonique ou un reste di(alkyl en $C_1$-$C_{24}$)amino, (acyl en $C_1$-$C_{24}$)-amino, (alkyl $C_1$-$C_{24}$)sulfate, (alkyl $C_1$-$C_{24}$)-sulfonate, (alkyl $C_1$-$C_{24}$)sulfite, (alkyl $C_1$-$C_{24}$)-sulfoxyde, (alkoxy $C_1$-$C_{24}$)carbonyle, mais $R^1$ et $R^2$ ne sont pas des atomes d'hydrogène simultanément,

$R^3$ représente un reste polyhydroxyalkyle de formule générale

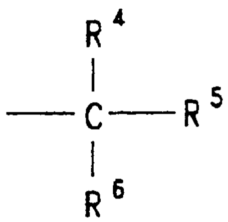

(II)

où

$R^4$ = $-CHOR^7)_n-H$,
$R^5$ = $-(CHOR^7)_m-H$,
$R^6$ = $-(CHOR^7)_o-H$ avec $n + m + o = 2-6$ et
$R^7$ = H ou un monosaccharide, lié par une liaison glycoside, de la famille des sucres réducteurs.

**2.** Procédé de préparation des imides de l'acide succinique de formule I comprenant les stades :

- introduction de l'aminopolyol de formule III

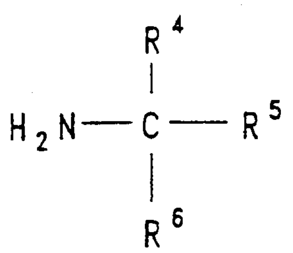

(III)

où $R^3$, $R^4$ et $R^5$ possèdent la signification donnée à la revendication 1, dans une suspension ou un solvant,
- ajour de l'acide carboxylique IIa et/ou de l'anhydride d'acide carboxylique IIb

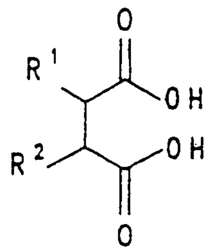

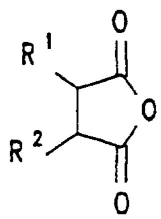

où $R^1$ et $R^2$ possèdent la signification donnée à la revendication 1, éventuellement en chauffant légèrement et
- chauffage de ce mélange à une température et pendant une durée telles que le mélange obtenu soit essentiellement débarrassé de l'agent de suspension ou du solvant, respectivement de l'eau réactionnelle, et que le mélange réactionnel présente une teneur en azote de base inférieure à 0,1% massique.

**3.** Procédé selon la revendication 2, caractérisé en ce qu'on utilise en tant qu'agent de suspension ou solvant l'eau, des alcools, de préférence des alcools aliphatiques inférieurs ou un mélange eau/alcool.

**4.** Procédé selon la revendication 2, caractérisé en ce qu'on réallse l'ajout à une température comprise entre 20 à 80°C, de préférence de 50 à 70°C.

**5.** Procédé selon la revendication 2, caractérisé en ce qu'on chauffe le mélange, en vue d'éliminer l'agent de suspension ou le solvant présents, a une température jusqu'à 160°C au maximum et ensuite éventuellement à une température jusqu'à 190°C au maximum.

**6.** Procédé selon la revendication 2, caractérisé en ce qu'on chauffe le mélange pendant une durée allant jusqu'à 3 heures.

**7.** Utilisation des imides de l'acide succinique de formule I selon la revendication 1 en tant que matière première pour détergents ou produit de protection contre la corrosion.

**8.** Solution aqueuse contenant des imides de l'acide succinique de formule I selon la revendication 1.

**9.** Solution aqueuse selon la revendication 8, caractérisée en ce qu'elle contient de 10 à 60% massiques d'une base

azotée, de préférence des amines tertiaires, et présente un pH supérieur à 7.

**10.** Utilisation de la solution aqueuse selon la revendication 8 en tant qu'agent de protection contre la corrosion.